# EUROPÄISCHE PATENTANMELDUNG

(11) **EP 0 902 002 A1**
(43) Veröffentlichungstag der Anmeldung: **17.03.1999**
(21) Anmeldenummer: 98116936.0
(22) Anmeldetag: 08.09.1998
(51) Int. Cl.: C07C 47/544, C07C 47/56, A61K 31/11

(54) **Neue Phthalaldehyd-Derivate, Verfahren zu deren Herstellung und deren Verwendung**

(30) Priorität: 12.09.1997 DE 19740080
(71) Anmelder: Hoechst Marion Roussel Deutschland GmbH, 65929 Frankfurt am Main (DE)
(72) Erfinder: Vértesy, Laszlo, Dr., 65817 Eppstein (DE); Kurz, Michael, Dr., 65719 Hofheim (DE); Schindler, Peter, Dr., 65812 Bad Soden (DE); Stump, Heike, DI., 61184 Karben (DE)

(57) **Zusammenfassung**

Die Erfindung betrifft neue Phthalaldehyd-Derivate, insbesondere Hericenal C, Verfahren zu deren Herstellung und die Verwendung von Phthalaldehyd-Derivaten zur Herstellung von Arzneimitteln zur prophylaktischen und therapeutischen Behandlung von Stoffwechselerkrankungen, insbesondere Glucose-Stoffwechselstörungen bzw. Stoffwechselerkrankungen des menschlichen Körpers. Die Erfindung betrifft insbesondere die Verwendung von Phthalaldehyd-Derivaten zur Herstellung von Arzneimitteln, die zur Behandlung von Diabetes mellitus eingesetzt werden können.

## Beschreibung

Die Erfindung betrifft neue Phthalaldehyd-Derivate, insbesondere Hericenal C, Verfahren zu deren Herstellung und die Verwendung von Phthalaldehyd-Derivaten zur Herstellung von Arzneimitteln zur prophylaktischen und therapeutischen Behandlung von Stoffwechselerkrankungen, insbesondere Glucose-Stoffwechselstörungen bzw. Glucose-Stoffwechselerkrankungen des menschlichen Körpers.
Die Erfindung betrifft insbesondere die Verwendung von Phthalaldehyd-Derivaten zur Herstellung von Arzneimitteln, die Zur Behandlung von Diabetes mellitus eingesetzt werden können.

Das Krankheitsbild des Diabetes mellitus ist durch erhöhte Blutzuckerwerte gekennzeichnet. Beim insulinpflichtigen oder Typ I Diabetes ist die Ursache das Absterben der insulinproduzierenden β-Zellen des Pankreas; die Behandlung erfolgt daher durch Insulingabe (Substitutionstherapie). Der nicht-insulinabhängige oder Typ II Diabetes ist dagegen durch eine verminderte Insulinwirkung auf Muskel-und Fettgewebe (Insulinresistenz) und eine gesteigerte Glucoseproduktion der Leber gekennzeichnet. Die von der Leber in das Blut abgegebene Glucose kann sowohl durch Abbau von Leber-Glycogen (Glycogenolyse) als auch durch Gluconeogenese gebildet werden. Glucose-6-Phosphat ist das gemeinsame Endprodukt sowohl der Glycogenolyse als auch der Gluconeogenese. Der terminale Schritt der hepatischen Freisetzung von Glucose aus Glucose-6-Phosphat wird von der Glucose-6-Phosphatase (EC 3.1.3.9) katalysiert.

Die Glucose-6-Phosphatase der Leber ist ein Multi-Enzym-Komplex, der wenigstens drei Wirkungseinheiten umfaßt: eine Glucose-6-Phosphat-Translocase (T1), eine Glucose-6-Phosphat-Phosphohydrolase sowie eine Phosphat / Pyrophosphat-Translocase (T2). Die Glucose-6-Phosphat-Translocase besitzt ein hohes Maß an Substrat-Spezifität; sie ist deshalb ein besonders geeignetes Target für die gezielte Hemmung der Glucosefreisetzung und damit zur Behandlung von z.B. Diabetes mellitus. Von den vielen natürlichen Zuckerphosphaten wird nur Glucose-6-Phosphat transloziert, wohingegen Phosphatasen wie z.B. Glucose-6-Phosphat-Phosphohydrase die organischen Phosphat-Ester mehrerer Substrate spalten.

In der Literatur wurden eine ganze Reihe relativ unspezifischer Glucose-6-Phosphat-Translocase-Inhibitoren beschrieben. Die ersten therapeutisch verwendbaren Inhibitoren wurden in den Europäischen Patentanmeldungen Nr. 93 114 260.8, Nr. 93 114 261.6 und Nr. 97 109 900.7 beschrieben.

Überraschenderweise wurde nun gefunden, daß aus der Kultur des Basidomyceten Hericium erinaceus Sekundärmetabolite, insbesondere Phthalaldehyd-Derivate, isoliert werden können, die die Glucose-6-Phosphat-Translocase spezifisch inhibieren.

Gegenstand der Erfindung sind Phthalaldehyd-Derivate der Formel I 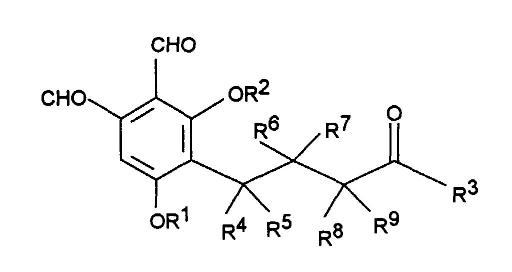 worin
- R¹ und R²: unabhängig voneinander Wasserstoff, C₁-C₄Alkyl, C₁-C₄Alkenyl oder C₁-C₄Acyl sind;
- R³: Wasserstoff, C₁-C₄Alkyl, C₁-C₄Alkenyl, OH, OR¹, SR¹, NR¹R² ist, wobei R¹ und R² wie oben definiert sind;
- R⁴ bis R⁹: unabhängig voneinander Wasserstoff oder Methyl bedeuten;
sowie die physiologisch verträglichen Salze der Verbindungen der Formel I.
C₁-C₄ Alkyl bedeutet lineare und verzweigte Alkylreste,
   beispielsweise -CH₃, -CH₂-CH₃, -(CH₂)₂-CH₃, -(CH₂)₃ - CH₃, -CH(CH₃)-CH₃, -CH(CH₂-CH₃)-CH₃, -CH₂-CH(CH₃)-CH₃;
C₁-C₄Alkenyl bedeutet lineare und verzweigte Alkenylreste
   beispielsweise -CH=CH₂, -CH₂-CH=CH₂, -(CH₂)₂-CH=CH₂, -CH=CH-CH₂, -C(CH₃)=CH-CH₂, -CH=C(CH₃)-CH₂, -CH=CH-CH₂-CH₃, -CH₂-CH=CH-CH₃
C₁-C₄Acyl bedeutet beispielsweise

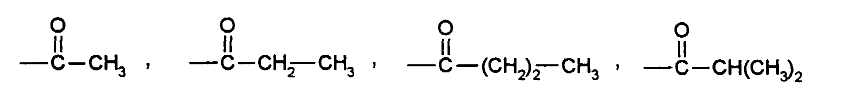

Unter physiologisch verträglichen Salzen werden in Wasser leicht lösliche, lösliche und wenig lösliche Verbindungen gemäß der Definition im "Deutschen Arzneibuch" (9. Ausgabe 1986, Amtliche Ausgabe, Deutscher Apotheker Verlag Stuttgart), Seite 19 verstanden.

Spezielle Ausführungsformen der Erfindung sind Verbindungen der Formel I in denen
R¹, R², R³ und R⁴ bis R⁹ wie oben definiert sind, mit der Maßgabe, daß in der mit R⁴ bis R⁹ substituierten Kohlenstoffkette jeweils nur einer der Substituenten R⁴ bis R⁹ Metyhl bedeutet.

Bevorzugte Ausführungsformen der Erfindung sind Verbindungen der Formel I, in denen
- R¹ und R²: Wasserstoff sind;
- R³: Wasserstoff, -OH oder C₁-C₄-Alkyl sind;
- R⁴ bis R⁹: Wasserstoff oder Methyl sind, mit der Maßgabe, daß in der mit R⁴ bis R⁹ substituierten Kohlenstoffkette jeweils nur einer der substituenten R⁴ bis R⁹ Methyl ist.

Eine besondere Ausführungsform der Erfindung (Phthalaldehyd-Derivat nach Formel I, wobei R¹ bis R⁸ Wasserstoff sind und R⁹ Methyl ist) ist die Verbindung Hericenal C. Hericenal C läßt sich beispielsweise aus Hericium erinaceus isolieren. Hericenal C hat die folgende Strukturformel:

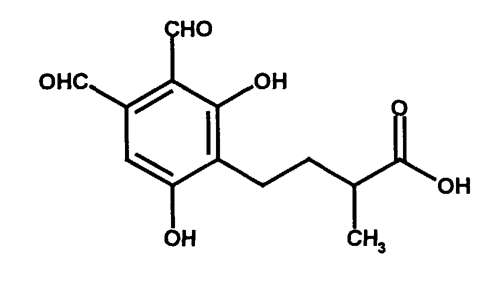

Gegenstand der Erfindung ist Hericenal C sowie dessen physiologisch verträgliche Salze (z.B. Deutsches Arzneibuch, 1986, Deutscher Apotheker Verlag Stuttgart, S. 19; Remington's Pharmaceutical Sciences, 1985, Mack Publishing Company, S. 1418 ff). Gegenstand der Erfindung sind auch die offensichtlichen chemischen Äquivalente von Hericenal C wie zum Beispiel dessen Ester, Säureamide, Ether, Acetale und/oder Halbacetale sowie Oxidationsprodukte. Chemische Äquivalente können beispielsweise unter bestimmten physiologischen Bedingungen in Hericenal C umgewandelt werden.

Chemische Äquivalente sind Verbindungen, die unter physiologischen Bedingungen in Hericenal C umgewandelte werden oder die in Form ihres chemischen Äquivalents die gleiche oder eine bessere physiologische Wirkung haben (d.h. spezifisch die Glucose-6-Phosphat-Translocase inhibieren). Chemische Äquivalente können in analoger Weise wie Hericenal C verwendet werden.

Weiterhin beinhaltet die vorliegende Erfindung Verfahren zur Herstellung der Verbindungen der Formel I, insbesondere von Hericenal C. Ein Verfahren zur Herstellung ist beispielsweise dadurch gekennzeichnet, daß der Mikroorganismus Hericium erinaceus, vorzugsweise Hericium erinaceus DSM 10600, in einem wäßrigen Medium kultiviert wird und die Zielverbindungen anschließend isoliert und gereinigt werden.

Der Mikroorganismus Hericium erinaceus DSM 10600 wurde am 20. März 1996 bei der Deutschen Sammlung von Mikroorganismen und Zellkulturen GmbH (DSMZ), Marschroderweg 1b, D-38124 Braunschweig, nach den Vorschriften des Budapester Vertrags hinterlegt.

Anstelle des Stammes DSM 10600 können auch dessen Mutanten und/oder Varianten eingesetzt werden, soweit sie Verbindungen der Formel I, insbesondere Hericenal C und/oder dessen chemische Äquivalente synthetisieren können. Solche Mutanten und/oder Varianten können in an sich bekannter Weise z.B. durch physikalische Mittel, beispielsweise Bestrahlung, wie mit Ultraviolett- und/oder Röntgenstrahlen und/oder chemische Mutagene, beispielsweise Ethylmethansulfonat (EMS), 2-Hydroxy-4-methoxy-benzophenon (MOB) und/oder N-Methyl-N'-nitro-N-nitrosoguanidin (MNNG) erzeugt werden. Mutanten und/oder Varianten können auch mit Hilfe von bekannten molekularbiologischen Methoden hergestellt werden.

Die im folgenden beschriebenen Fermentationsbedingungen gelten für Hericium erinaceus und DSM 10600. Die Fermentation kann z.B. im Labormaßstab (Milliliter-und Litermaßstab) oder im industriellen Maßstab (Kubikmetermaßstab) erfolgen.

In einer Nährlösung, die eine Kohlenstoffquelle und eine Stickstoffquelle sowie die üblichen anorganischen Salze enthält, produziert Hericium erinaceus (H. erinaceus), insbesondere Hericium erinaceus DSM 10600, die Verbindung der Formel I, beispielsweise Hericenal C.

Als bevorzugte Kohlenstoffquellen für die aerobe Fermentation eignen sich assimilierbare Kohlenhydrate und Zuckeralkohole, wie z.B. Glukose, Laktose oder D-Mannit sowie kohlenhydrathaltige Naturprodukte, wie z. B. Malzextrakt. Als stickstoffhaltige Nährstoffe kommen z.B. Aminosäuren, Peptide und Proteine sowie deren Abbauprodukte, wie Peptone oder Tryptone, ferner Fleischextrakte, gemahlene Samen, beispielsweise von Mais, Weizen, Bohnen, Soja oder der Baumwollpflanze, Destillationsrückstände der Alkoholherstellung, Fleischmehle oder Hefeextrakte, aber auch Ammoniumsalze und Nitrate in Betracht. An anorganischen Salzen kann die Nährlösung beispielsweise Chloride, Carbonate, Sulfate oder Phosphate der Alkali- und/oder Erdalkalimetalle und/oder Übergangsmetalle, z.B. Eisen, Zink, Kobalt und/oder Mangan enthalten.

Die Bildung der Verbindungen der Formel I, insbesondere von Hericenal C mit H. erinaceus, bevorzugt DSM 10600 verläuft besonders gut in einer Nährlösung, die 0,1 bis 2 %, bevorzugt 0,5 - 1%, Cornstep, 0,5 bis 10%, bevorzugt 1 bis 5 % Tomatenpaste, 0,1 bis 5 %, bevorzugt 0,5 bis 3 % Hafermehl 0,1 bis 5 %, bevorzugt 0,5 bis 3 % Glucose sowie gegebenenfalls Spurenelemente enthält (% Angaben sind jeweils bezogen auf das Gewicht der gesamten Nährlösung). Die Spurenelementlösung kann beispielsweise folgende Zusammensetzung aufweisen:

| | | |
|---|---|---|
| FeSO₄ x 7 H₂O | 0,01-1%, bevorzugt | 0,05 - 0,2 % und/oder |
| MuSO₄ x 1 H₂O | 0,01-1%, bevorzugt | 0,05 - 0,2 % und/oder |
| CuCl₂ x 2 H₂O | 0,0005-0,01%, bevorzugt | 0,001 - 0,005 % und/oder |
| CaCl₂ x 2 H₂O | 0,001-0,1%, bevorzugt | 0,005 - 0,05 % und/oder |
| H₃BO₃ | 0,005-0,01%, bevorzugt | 0,002 - 0,0075 % und/oder |
| (NH₄)₆ Mo₇ O₂₄ x 4 H₂O | 0,0005-0,01%, bevorzugt | 0,001 - 0,005 % und/oder |
| ZnSO₄ x 7 H₂O | 0,001-0,1%, bevorzugt | 0,001 - 0,05 %. |

Die Kultivierung erfolgt vorzugsweise aerob, also beispielsweise submers unter Schütteln und/oder Rühren und/oder in Fermentern, gegebenenfalls unter Einführen von Luft und/oder Sauerstoff. Sie wird beispielsweise in einem Temperaturbereich von 15 bis 35°C, vorzugsweise von 20 bis 30°C, insbesondere von 23 bis 28°C durchgeführt. Der pH-Wert der Kulturlösung liegt beispielsweise zwischen pH 1 und pH 8, vorteilhaft zwischen pH 2,5 und pH 4,5. Der Pilz H. erinaceus wird unter diesen Bedingungen über einen Zeitraum von 100 bis 300 Stunden kultiviert, bevorzugt 120 bis 168 Stunden lang.

Eine Ausführungsform der Erfindung beinhaltet, daß die Kultivierung in mehreren Stufen erfolgt, d.h. es werden zunächst eine oder mehrere Vorkulturen in einem flüssigen Nährmedium hergestellt, die dann in das eigentliche Produktionsmedium, die Hauptkultur, beispielsweise im Volumenverhaltnis 1:10, überimpft werden. Die Vorkultur wird z.B. dadurch erhalten, daß Mycel in eine Nährlösung überimpft werden und diese etwa 100 bis 200 Stunden, bevorzugt 120 bis 168 Stunden, wachsen gelassen werden. Das Mycel kann beispielsweise erhalten werden, indem der Stamm 7 bis 40 Tage, bevorzugt 10 bis 20 Tage, auf einem festen oder flüssigen Nährboden, beispielsweise Hefe-Malz-Agar oder Kartoffel-Dextrose-Agar wächst.

Der Fermentationsverlauf kann beispielsweise jeweils anhand des pH-Wertes der Kultur oder des Mycelvolumens sowie durch chromatographische Methoden, wie z. B. Dünnschichtchromatographie oder High Pressure Liquid Chromatography (HPLC) überwacht werden. Verbindungen der Formel I, insbesondere Hericenal C sind überwiegend im Kulturfiltrat enthalten. Für die Dünnschichtchromatographie werden als Laufmittel beispielsweise Ethylacetat/Methanol/Wasser-Mischungen in dem Fachmann bekannten Konzentrationen, angewandt. Die Detektion bei der dünnschichtchromatographischen Auftrennung kann beispielswiese durch UV-Löschung bzw. durch Verwenden von Färbereagentien wie Anisaldehyd, Tetrazolblau oder Orcin erfolgen.

Die Erfindung beinhaltet auch die chemische Synthese von Verbindungen der Formel I, insbesondere von Hericenal C und/oder der physiologisch verträglichen Salze derselben.

Die Isolierung eines Phthalaldehyd-Derivats der Formel I, insbesondere von Hericenal C und/oder dessen chemischen Äquivalenten aus dem jeweiligen Kulturmedium oder dem Reaktionsgemisch kann nach bekannten Methoden unter Berücksichtigung der chemischen, physikalischen und/oder biologischen Eigenschaften der Verbindungen erfolgen. Es können hierfür z. B. das Adsorptions-, Ionenaustausch- oder Gelfiltrationsverfahren angewendet werden.

Die Verbindungen der Formel I, insbesondere Hericenal C können sowohl im Mycel als auch im Kulturfiltrat vorkommen, gewöhnlich befindet sich die Hauptmenge im Kulturfiltrat. Es ist deshalb zweckmäßig, die Zellmasse durch Filtration und/oder Zentrifugation vom Filtrat zu trennen. Das Filtrat kann dann lyophilisiert und das Lyophilisat mit einem Lösungmittel extrahiert werden, beispielsweise mit Methanol oder Acetonitril oder 1-Butanol. Analog können Verbindungen der Formel I, insbesondere Hericenal C aus dem Mycel extrahiert werden. Die Extraktionen können über einen weiten pH-Bereich durchgeführt werden, besonders geeignet ist die Extraktion im pH-Bereich von pH 3.0 - pH 7.5.
Eine Methode der Isolierung ist die Lösungs-Verteilung in an sich bekannter Weise. Eine weitere Methode der Reinigung bietet die Chromatographie an Adsorptionsharzen wie z.B. an Diaion® HP-20 (Mitsubishi Casei Corp., Tokyo, Japan) an Amberlite® XAD 7 (Rohm und Haas, USA) an Amberchrom® CG (Toso Haas, Philadelphia, USA) oder an ähnlichen Trägern. Geeignet sind darüberhinaus zahlreiche reversed-phase Träger, z.B. RP-18, wie sie in der Hochdruckflüssigkeits-Chromatographie allgemein verwendet werden. Eine weitere Reinigungsmöglichkeit besteht in der Verwendung sogenannter normal-phasen Träger wie z. B. Kieselgel oder Aluminiumoxid oder anderen Trägern in an sich bekannter Weise. Geeignet zur Elution sind verschiedene Lösungsmittel, insbesondere Lösungsmittelgemische beispielsweise Chloroform/Methanol- oder Ethylacetat-Methanol-Gemische.

Ein alternatives Verfahren zur Isolierung von Phthalaldehyd-Derivaten der Formel I, insbesondere von Hericenal C und/oder dessen chemischen Äquivalenten bietet die Verwendung sog. Molekularsiebe, wie z.B. Fractogel® TSK HW-40, Sephadex® LH-20 in bekannter Weise.

Es ist darüberhinaus möglich diese Verbindungen aus angereichertem Material (aus dem Filtrat oder dem Mycel) durch Kristallisation zu gewinnen und/oder zu reinigen. Geeignet hierzu sind z.B. organische Lösungsmittel und deren Gemische, die gegebenenfalls Wasser enthalten können. Wasserhaltige Gemische können gegebenenfalls Basen, wie z. B. Natronlauge oder Säuren, wie z.B. Trifluoressigsäure, Schwefelsäure, Salzsäure, Ameisensäure enthalten.

Die Erfindung beinhaltet weiterhin, daß Verbindungen der Formel I, insbesondere Hericenal C, die entweder aus H, erinaceus isoliert oder chemisch hergestellt wurden unter Anwendung bekannter Verfahren in ihre physiologisch verträglichen Salze überführt werden.

Die Erfindung betrifft die Verwendung eines Phthalaldehyd-Derivats zur Inhibition der Glucose-6-Phosphat-Translocase. Vorzugsweise betrifft die Erfindung die Verwendung eines Phthalaldehyd-Derivats zur Inhibition der Glucose-6-Phosphat-Translocase, wobei das Phthalaldehyd-Derivat die Formel II hat, wobei ein Phthalaldehyd-Derivat der Formel II 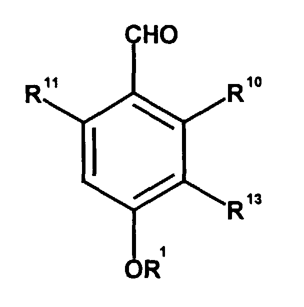 ist, worin
- entweder: R¹⁰ oder R¹¹ - CHO ist und der jeweils andere Rest R¹⁰ oder R¹¹ - OR² ist;
- R₁ und R₂: unabhängig voneinander Wasserstoff, C₁-C₄ Alkyl, C₁-C₄ Alkenyl oder C₁-C₄ Acyl sind;
- R¹³: C₁-C₄Alkyl, -C(R⁴R⁵)-C(R⁶R⁷)-C(R⁸R⁹)-C(O)R³, oder C(R¹⁴R¹⁵)-C(R¹⁶R¹⁷)-C(R¹⁸R¹⁹)-C(R²⁰R²¹)-C(R²²R²³)-C(O)R³
ist, wobei
- R³: Wasserstoff, -OH oder C₁-C₄Alkyl ist;
- R⁴ bis R⁹: Wasserstoff oder Methyl sind, mit der Maßgabe, daß in der mit R⁴ bis R⁹ substituierten Kohlenstoffkette jeweils nur einer der substituierten R⁴ bis R⁹ Methyl ist;
- R¹⁴ bis R²³: Wasserstoff oder Methyl bedeuten, wobei zwei sich an benachbarten Kohlenstoffatomen befindende R¹⁴ bis R²³ zusammen mit einer C-C-Einfachbindung für eine C-C-Doppelbindung stehen können, mit der Maßgabe, daß in der mit R¹⁴ bis R²³ substituierten Kohlenstoffkette jeweils nur einer der Substituenten R¹⁴ bis R²³ Methyl bedeutet und jeweils nur ein benachbartes Paar dieser Substituenten zu einer C-C-Doppelbindung beiträgt, vorzugsweise bedeutet R¹⁸ Methyl, besonders bevorzugt bedeuten entweder R¹⁶ oder R¹⁷ und R¹⁸ oder R¹⁹ zusammen mit der zwischen den von ihnen substituierten Kohlenstoffatomen liegenden C-C-Einfachbindung eine C-C-Doppelbindung oder R¹⁸ oder R¹⁹ und R²⁰ oder R²¹ zusammen mit der zwischen den von ihnen substituierten Kohlenstoffatomen liegenden C-C-Einfachbindung eine C-C-Doppelbindung.

Insbesondere betrifft die Erfindung die Verwendung eines Phthalaldehyd-Derivats der Formel II zur Inhibition der Glucose-6-Phosphat-Translocase, wobei
a) R¹⁰ - CHO, R¹¹ -OH, R¹³ -CH₃ und R¹ Wasserstoff ist (Flavipin); oder
b) R¹⁰ -OH, R¹¹ -CHO, R¹³ -C(R¹⁴R¹⁵)-C(R¹⁶R¹⁷)-C(R¹⁸R¹⁹)-C(R²⁰R²¹)-C(R²²R²³)-C(O)R³, R¹ Wasserstoff und R³ -OH sind, wobei vorzugsweise R¹⁴, R¹⁵, R¹⁶, R²⁰, R²¹, R²² und R²³ Wasserstoff, R¹⁸ Methyl und R¹⁷ und R¹⁹ zusammen eine Doppelbindung sind (Hericenal A); oder
c) R¹⁰ -OH, R¹¹ -CHO, R¹³ -C(R¹⁴R¹⁵)-C(R¹⁶R¹⁷)-C(R¹⁸R¹⁹)-C(R²⁰R²¹)-C-(R²²R²³)-C(O)R³, R¹ Wasserstoff, R²⁰ R²² und R²³ Wasserstoff, R¹⁸ Methyl und R¹⁹ und R²¹ zusammen eine Doppelbindung sind (Hericenal B); oder
d) R¹⁰ -OH, R¹¹ -CHO, R¹³ -C(R⁴R⁵)-C-(R⁶R⁷)-C-(R⁸R⁹)-C(O)R³, R¹ Wasserstoff und R³ -OH, sind, wobei vorzugsweise R⁴, R⁵, R⁶, R⁷ und R⁹ Wasserstoff sind und R⁸ Methyl ist (Hericenal C).

Überraschend wurde gefunden, daß Hericenal C das Enzym Glucose-6-Phosphat-Translocase spezifisch hemmt. Für Hericenal C wurde ein IC₅₀-Wert von 8 µg/ml bestimmt. Darüberhinaus wurde überraschend gefunden, daß auch andere Phthalaldehyd-Derivate die Glucose-6-Phosphat-Translocase spezifisch hemmen. Insbesondere wurden für die Verbindungen Hericenal A und Hericenal B mit 8,6 µg/ml ähnliche IC₅₀-Werte bestimmt. Für Flavipin wurde ein IC₅₀-Wert von 15 µg/ml bestimmt.

Zur Bestimmung der IC₅₀-Werte wurde die Glucose-6-Phosphat-Translocase Aktivität in Abhängigkeit vom Logarithmus der Konzentration des zu testenden Phthalaldehyd-Derivats aufgetragen. Der IC₅₀-Wert ist die Konzentration des zu testenden Phthalaldehyd-Derivats, bei der die Glucose-6-Phosphat-Translocase Aktivität zu 50 % gehemmt ist.

Die aufgeführten Verbindungen haben in dem angegebenen Konzentrationsbereich keinen oder einen nur vernachlässigbaren Effekt auf die Aktivität der Phosphat / Pyrophosphat-Translocase sowie Glucose-6-Phosphat-Phosphohydrolase.

Hericenal A und Hericenal B sind in der Internationalen Patentanmeldung mit dem Aktenzeichen PCT/EP97/01504 beschrieben. Diese Verbindungen weisen folgende Strukturformeln auf:

### Hericenal A:

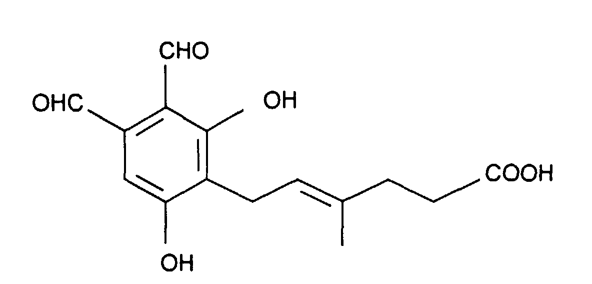

### Hericenal B:

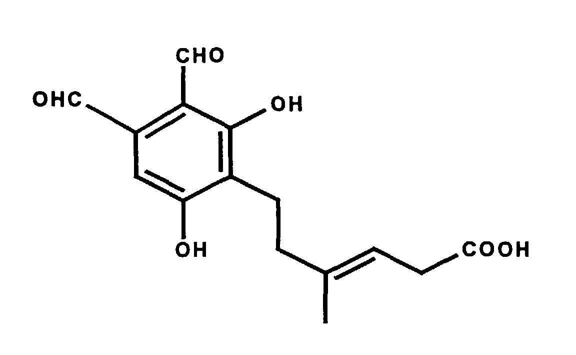

Flavipin (3,4,5-Trihydroxy-6-methyl-o-phthalaldehyd) wurde aus Aspergillus terreus isoliert und erstmals von H. Raistrick et al. (Biochem. J. (1956), **63**, Seite 395) beschrieben.

Flavipin weist folgende Strukturformel auf:

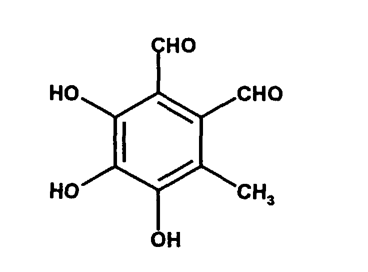

Die Erfindung betrifft Phthalaldehyd-Derivate, die spezifisch die Glucose-6-Phosphat-Translocase modulieren, insbesondere Phthalaldehyd-Derivate, die die Glucose-6-Phosphat-Translocase ganz oder teilweise inhibieren sowie die Verwendung derselben.
Eine Ausführungsform der Erfindung betrifft Phthalaldehyd-Derivate, die die Glucose-6-Phosphat-Translocase mit einem IC₅₀-Wert kleiner als 25 mg/ml, vorzugsweise kleiner als 20 mg/ml, besonders bevorzugt kleiner oder gleich 15 mg/ml inhibieren. Vorzugsweise werden dabei die IC₅₀-Werte wie in Beispiel 5 beschrieben, bestimmt.

Die Erfindung betrifft besonders die Verwendung von Verbindungen der der Formel II und/oder der Formel I, insbesondere von Hericenal C, Hericenal A, Hericenal B und Flavipin zur Inhibition der Glucose-6-Phosphat-Translocase.

Die Erfindung betrifft weiterhin die Verwendung von Phthalaldehyd-Derivaten, insbesondere von Verbindungen der Formel II oder Verbindungen derFormel I, z.B. von Hericenal C, A, B und Flavipin zur Herstellung von Arzneimitteln, die zur Prophylaxe und/oder Behandlung von Krankheiten, die durch Hemmung der Glucose-6-Phosphat-Translocase positiv beeinfluß werden können, benutzt werden können.

Insbesondere betrifft das die Herstellung von Arzneimitteln, die zur Behandlung von Krankheiten, die mit überhöhter Blutglucose-Konzentration einhergehen, verwendet werden können.

Eine spezielle Ausführungsform der Erfindung betrifft die Verwendung von Phthalaldehyd-Derivaten zur Herstellung von Arzneimitteln, die zur Behandlung von Diabetes, besonders von Diabetes mellitus, insbesondere von Diabetes mellitus Typ II verwendet werden können.

Die Erfindung betrifft insbesondere die Verwendung von Hericenal C, Hericenal A, Hericenal B und/oder Flavipin und/oder deren chemischen Äquivalenten bzw. den physiologisch verträglichen Salzen derselben zur Herstellung von Arzneimitteln zur Behandlung von Diabetes mellitus, insbesondere von Diabetes mellitus Typ II.

Die Arzneimittel können als Wirkstoff wahlweise nur ein Phthalaldehyd-Derivat oder aber eine Mischung zweier oder mehrerer verschiedener Phthalaldehyd-Derivate enthalten.

Die Arzneimittel können nach bekannten Verfahren hergestellt werden. Zur Herstellung können die erfindungsgemäßen und pharmakologisch wirksamen Phthalaldehyd-Derivate ( = Wirkstoffe ) entweder als solche, oder vorzugsweise in Kombination mit geeigneten pharmazeutischen Hilfsstoffen in Form von Dragees, Tabletten, Kapseln, Suppositorien, Emulsionen Suspensionen, Pulvern, Lösungen oder Präparaten mit protrahierender Wirkstoff-Freigabe, eingesetzt werden, wobei der Wirkstoffgehalt vorteilhafterweise 0,1 bis 95% betragen kann.

Die Arzneimittel können ein oder mehrere Phthalaldehyd-Derivate und ein oder mehrere pharmazeutische Hilfsstoffe enthalten.

Die vorliegende Erfindung betrifft die verwendung von Hericium erinaceus DSM 10600 sowie dessen Mutanten und Varianten zur Herstellung von Verbindungen der Formel I, insbesondere von Hericenal C bzw. dessen chemischen Äquivalenten.

In den nicht-limitierenden Beispielen wird die Erfindung weiter erläutert. Prozentangaben beziehen sich auf das Gewicht, Mischungsverhältnisse bei Flüssigkeiten beziehen sich auf das Volumen, wenn keine anderen Angaben gemacht wurden.

### Beispiele

### Beispiel 1:

### a) Herstellung von Mycel des Produzentenstammes H. erinaceus.

100 ml Nährlösung (20 g Malzextrakt, 2 g Hefeextrakt, 10g Glucose, 0,5 g (NH₄)₂HPO₄ in 1 L Leitungswasser, pH-Wert vor der Sterilisation 6,0) werden in einem sterilen 500 ml Erlenmeyerkolben mit dem H. erinaceus beimpft und 240 Stunden bei 25°C und 140 UpM (Umdrehungen pro Minute) auf einer rotierenden Schüttelmaschine inkubiert. Anschließend werden 20 ml Kulturflüssigkeit in einem sterilen 500 ml Erlenmeyerkolben mit dem Nährboden [Kartoffelinfus 4,0 g/l (Infus aus 200 g Kartoffel in 1000 ml H₂O), 20,0 g D-Glucose, pH 5,6 vor der Sterilisation] dem zusätzlich 15 g Agar/l zur Verfestigung zugegeben wurde, gleichmäßig verteilt und dekantiert. Die Kulturen werden 10 bis 21 Tage bei 25°C inkubiert. Das nach dieser Zeit entstandene Mycel eines Kolbens wird mit einer sterilen Impfnadel abgenommen, sofort weiterverwendet oder bei -4°C in Wasser aufbewahrt.

### b) Herstellung einer Vorkultur des Produzentenstammes

Ein steriler 500 ml Erlenmeyerkolben mit 100 ml der unter a) beschriebenen Nährlösung wird mit einer auf einem Schrägröhrchen gewachsenen Kultur oder mit einem 1 Quadratzentimeter großen Agarstück angeimpft und auf einer Schüttelmaschine bei 140 UpM und 25°C inkubiert. Die maximale Produktion einer Verbindung der Formel I ist nach ca. 168 Stunden erreicht. Zum Animpfen (500 ml in 200 ml Erlenmeyer) genügt eine 168 Stunden alte Submerskultur (Animpfmenge ca. 5 %) aus der gleichen Nährlösung.

### Beispiel 2:

### Herstellung der Hericenale.

Ein 2000 ml Erlenmeyer Schüttelkolben wird unter folgenden Bedingungen betrieben:

| | |
|---|---|
| 5 g/L Cornstep, | 10 g / L Glucose, |
| 4 g/L Tomatenpaste, | 10 ml / L Spurenelementlösung, |
| 10 g/L Hafermehl, | |
| pH 6,8 (vor der Sterilisation) | |

Der pH-Wert wird mit wäßriger 2 N NaOH oder HCl eingestellt.
- Inkubationszeit:: 168 Stunden
- Inkubationstemperatur:: 25°C
- Schüttelgeschwindigkeit:: 140 Rpm
der Spurenelementlösung (Zusammensetzung in %):

| | |
|---|---|
| FeSO₄ x 7 H₂O | 0,100 |
| MnSO₄ x H₂O | 0,100 |
| CuCl₂ x 2 H₂O | 0,0025 |
| CaCl₂ x 2 H₂O | 0,010 |
| H₃BO₃ | 0,0056 |
| (NH₄)₆Mo₇O₂₄ x 4 H₂O | 0,0019 |
| ZnSO₄ x 7 H₂O | 0,020 |

### Beispiel 3:

### Isolierung der Hericenale.

Die nach Beispiel 2 gewonnene Kulturlösung wird abzentrifugiert und die Zellmasse wird gefriergetrocknet. Das klare Kulturfiltrat wird an Mitsubishi Diaion® CHP20 adsorbiert (Säulenmaße 30 x 6 cm) und mit einem Isopropanol Gradienten 0 - 70 % Isopropanol eluiert (Fluß 50 ml/min). Die die Hericenale enthaltenden Fraktionen werden vereinigt, im Vakuum bis auf die Wasserphase eingeengt und lyophilisiert.

### Beispiel 4:

### Reinigung der Hericenale (Hericenal A, Hericenal B, Hericenal C):

270 mg des in Beispiel 3 erhaltenen Rohgemisches werden in 20 % wäßrigem Acetonitril gelöst und zunächst an einer Säule mit Nucleosil® 100-12 C₁₈ mit einem Gradienten von 25 - 40 % Acetonitril in Wasser mit 0.05 % Trifluoressigsäure (Säulenmaße 250 x 32 mm, Fluß 50 ml/min) chromatographiert. Die Fraktionen, die die Hericenale enthalten werden vereinigt, im Vakuum konzentriert und an Nucleosil® 100-10 C₁₈ AB mit dem gleichen Gradienten endgereinigt (Säulenmaße 250 x 20 mm, Fluß 25 ml/min). Nach 18 Minuten wird Hericenal C von der Säule eluiert, nach 20,2 Minuten Hericenal A und B. Nach Konzentration der Fraktionen und Lyophilisation werden 12 mg Hericenal C sowie 20mg eines Gemisches aus Hercenal A und Hericenal B gewonnen. Das Gemisch von Hericenal A und B wird durch Säulenchromatographie an Kieselgel 60 (Merck, 0,015 - 0,040 mm, Säulenvolumen 5 ml) mit Dichlormethan /n-Butanol 20:1 als Eluens in die Einzelkomponenten getrennt.
Durch hochauflösende Massenspektrometrie des Hericenal C wird die Molmasse 266,078 ermittelt, entsprechend C₁₃H₁₄O₆. Die NMR-Signale sind in Tabelle 1 aufgeführt.

Die physikalisch-chemischen sowie spektroskopischen Eigenschaften von Hericenal C lassen sich wie folgt zusammenfassen:
- Aussehen:: hellgelbe, in Alkoholen und anderen polaren organischen Lösungsmitteln lösliche Substanz. Die Verbindung ist stabil in neutralem und mild saurem Milieu (vorzugsweise > pH 2), jedoch unbeständig in stark alkalischer Lösung (beispielsweise > pH 8).
- Summenformel:: C₁₃H₁₄O₆
- Molekulargewicht:: 266 g/mol
- Ultraviolet.Absorbtions-Maxima:: 250nm, 315nm Schulter bei 340nm (in Methanol)

### Beispiel 5:

### Glukose-6-Phosphat-Translocase Test

Material: Glukose-6-phosphat, Mannose-6-phosphat, Triton X-100™ und HEPES von Sigma Chemicals Co. (St. Louis, MO, USA); Saccharose und Ammoniumheptamolybdat von Merck (Darmstadt, Deutschland).

### a) Herstellung der Mikrosomen

Mikrosomen aus der Leber wurden, wie unter Nordlie, R.C.; Arion, W.J. Methods Enzymol. 1966, 9, 619-625 beschrieben, hergestellt. Der Proteingehalt der mikrosomalen Fraktion wurde nach der Bicinchoninicsäure Methode, wie in Smith, P.K.; Krohn, R.I.; Hermanson, G.T.; Mallia, A.K.; Gartner, F.H.; Provenzano, M.D.; Fujimoto, E.K.; Goeke, N.M.; Olson, B.J.; Klenk, D.C. Measurement of protein using bicinchoninic acid. Anal. Biochem. 1985, 150, 76-85 beschrieben, mit BSA (bovine serum albumin) als Standard bestimmt. Die Qualität (Intaktheit) der Mikrosomen wurde mit Mannose-6-phosphat, wie in Arion, W.J. Measurement of intactness of rat liver endoplasmic reticulum. Methods Enzymol. 1989, 174, 58-67 beschrieben bestimmt. Die größte Mannose-6-phosphat Aktivität zeigten vollständig zerstörte Mikrosomen, die bei 4°C aus Mikrosomenlösungen mit einem Proteingehalt von 10 mg Protein/ml Triton X-100™ (optimale Konzentration (0.3-0.4 %) hergestellt wurden. Es wurden Mikrosomen mit einer Intaktheit größer als 96 % verwendet.

### b) Bestimmung der Glukose-6-Phosphatase Aktivität

Die Glucose-6-Phosphatase Aktivität wurde mit Hilfe eines modifizierten Assays nach Arion (1989) Methods Enzymol. 1989, 174, 58-67 sowohl für unbehandelte als auch für Mikrosomen mit Triton X-100™ (siehe a)) durchgeführt. Als Assaypuffer wurde 250 mM Sucrose, 50 mM HEPES pH 7.0 verwendet. Dieser wurde mit 1 mM Glukose-6-phosphat, 1 mM der zu testenden Substanz (Hericenal A, Hericenal B, Hericenal C, Flavipin) versetzt. Zu je 100 µl Assaypuffer wurden 100 µg mikrosomales Protein gegeben, wodurch die Reaktion gestartet wurde. Die Reaktion wurde nach 10 min (für unbehandelte Mikrosomen) bzw. 5 min (für zerstörte Mikrosomen) durch Zugabe von 150 µl des Farbstoffs beendet. Die Mikrotiterplatte wurde für 30 min bei 37°C inkubiert und die Extinktion bei 630 nm bestimmt. Aus den gemessenen Extinktionen wurde die Inhibition der Glukose-6-phosphat Translocase mit Hilfe des Programms Turbo Basic ™ bestimmt.

### c) Bestimmung der IC₅₀-Werte

Der Assay wurde in Mikrotiterplatten in 100 µl Assaypuffer (250 mM Sucrose, 50 mM HEPES) pH 7 mit je 1 mM Glucose-6-phosphat und der zu testenden Substanz durchgeführt. Die Reaktion wurde durch Zugabe von 5 µg mikrosomalem Protein gestartet. Die Reaktion wurde wie unter b) beschrieben, nach 10 min (für unbehandelte Mikrosomen) bzw. 6 min (für zerstörte Mikrosomen) gestoppt und die Extinktion bei 820 nm bestimmt.

### d) Analyse

Die IC₅₀ Werte wurden mit Hilfe der Michaelis-Menten Gleichung für kompetitive Inhibitoren bestimmt ["Natürliche Enzyminhibitoren", Rosemarie Vogel, Georg Thieme Verlag, Stuttgart N.Y, 1984, S. 5 bis 7].

### e) Herstellung der zu testenden Substanzen

Stammlösungen der zu testenden Substanzen (80 mM) in Methanol wurden hergestellt und mit Assaypuffer (250 mM Sucrose, 50 mM HEPES, pH 7.0) bis zu einer Endkonzentration von 1 mM (im Assay) verdünnt.

**Tabelle 1**

| ¹H- und ¹³C-NMR-Signale von Hericenal C Chemische Verschiebungen von Hericenal C in DMSO_{d6} bei 300 K. | | | | | |
|---|---|---|---|---|---|
| | ^{**1**}**H** | ^{**13**}**C** | | ^{**1**}**H** | ^{**13**}**C** |
| *1* | - | 163.00 | *5-OH* | 11.273 | - |
| *1-OH* | 12.718 | - | *6* | - | 120.29 |
| *2* | - | 111.27 | *7* | 2.615 | 19.97 |
| *2-CHO* | 10.563 | 194.69 | *8* | 1.792/1.494 | 31.56 |
| *3* | - | 136.35 | *9* | 2.302 | 38.62 |
| *3-CHO* | 10.186 | 192.86 | *9-Methyl* | 1.105 | 16.78 |
| *4* | 7.039 | 113.40 | *10* | ∼11.8 | 177.13 |
| *5* | - | 162.32 | | | |

**Tabelle 2**

| Für Hericenal C und weitere Phthalaldehyd-Derivate sind folgende IC₅₀-Werte für die Inhibition der Glukose-6-Phosphat-Translocase gefunden worden: | |
|---|---|
| Hericenal C: | IC₅₀ = 8 µg / ml |
| Hericenal A | IC₅₀ = 8,6 µg / ml |
| Hericenal B | IC₅₀ = 8,6 µg / ml |
| Flavipin | IC₅₀ = 15 µg / ml |

## Patentansprüche

1. Verwendung von Phthalaldehyd-Derivaten zur Inhibition der Glucose-6-Phosphat-Translocase.

2. Verwendung von Phthalaldehyd-Derivaten nach Anspruch 1 zur Herstellung von Arzneimitteln, die zur Behandlung von Krankheiten verwendet werden können, die mit einer erhöhten Blutglucose-Konzentration einhergehen.

3. Verwendung von Phthalaldehyd-Derivaten nach einem oder mehreren der Ansprüche 1 und 2 zur Herstellung von Arzneimitteln, die zur Behandlung von Diabetes mellitus verwendet werden können.

4. Verwendung von Phthalaldehyd-Derivaten der Formel I 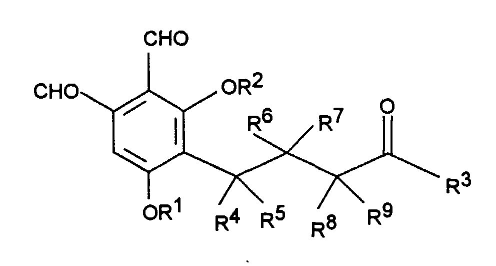 worin
R¹ und R² unabhängig voneinander Wasserstoff, C₁-C₄Alkyl, C₁-C₄ Alkenyl oder C₁-C₄Acyl sind;
R³ Wasserstoff, C₁-C₄Alkyl, C₁-C₄Alkenyl, OH, OR¹, SR¹ oder NR¹R² ist,
wobei R¹ und R² wie oben definiert sind;
R⁴ bis R⁹ unabhängig voneinander Wasserstoff oder Methyl bedeuten;
sowie der physiologisch verträglichen Salze der Verbindungen der Formel I
gemäß einem oder mehreren der Ansprüche 1 bis 3.

5. Verwendung von Hericenal A, Hericenal B und/oder Flavipin sowie deren physiologisch verträglichen Salze gemäß einem oder mehreren der Ansprüche 1 bis 3.

6. Phthalaldehyd-Derivate der Formel I 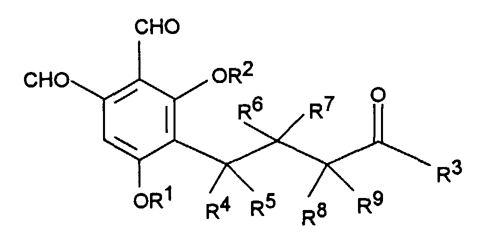 worin
R¹ und R² unabhängig voneinander Wasserstoff, C₁-C₄Alkyl, C₁-C₄ Alkenyl oder C₁-C₄Acyl sind;
R³ Wasserstoff, C₁-C₄Alkyl, C₁-C₄Alkenyl, OH, OR¹, SR¹ oder NR¹R² ist, wobei R¹ und R² wie oben definiert sind;
R⁴ bis R⁹ unabhängig voneinander Wasserstoff oder Methyl bedeuten;
sowie die physiologisch verträglichen Salze der Verbindungen der Formel I.

7. Phthalaldehyd-Derivat der Formel I nach Anspruch 6, worin R¹ bis R⁸ Wasserstoff sind und R⁹ Methyl ist sowie dessen physiologisch verträgliche Salze.

8. Verfahren zur Herstellung von Phthalaldehyd-Derivaten der Formel I nach einem oder mehreren der Ansprüche 6 und 7, wobei ein mikrobiologisches oder ein chemisches Verfahren verwendet wird.

9. Verfahren zur Herstellung nach Anspruch 8, dadurch gekennzeichnet, daß der Basidomycetenstamm Hericium erinaceus verwendet wird.

10. Verfahren zur Herstellung nach Anspruch 8, dadurch gekennzeichnet, daß der DSM 10600 verwendet wird.
